# EUROPEAN PATENT APPLICATION

(11) **EP 3 736 574 A1**
(43) Date of publication of application: **11.11.2020**
(21) Application number: 19172905.2
(22) Date of filing: 07.05.2019
(51) Int. Cl.: G01N 33/68, A61K 38/00

(54) **A FORMULATION COMPRISING AN ISOTOPE LABELED FUSION POLYPEPTIDE**

(71) Applicant: Atlas Antibodies AB, 168 69 Bromma (SE)
(72) Inventor: UTTERBÄCK, Marie, 182 32 Danderyd (SE); BOSTRÖM, Tove, 115 44 Stockholm (SE)
(74) Representative: Kransell & Wennborg KB

(57) **Abstract**

The present disclosure relates to an aqueous formulation comprising an isotope labeled fusion polypeptide that provides improvements with respect to storage and handling as well as assuring the quality of the fusion polypeptide for use as an internal standard in mass spectrometric based assays. The present disclosure also relates to a method for lyophilizing the aqueous formulation and to a method for determining the absolute amount of a target protein.

## Description

### TECHNICAL FIELD

The present disclosure generally relates to an aqueous formulation comprising an isotope labeled fusion polypeptide. The present disclosure also relates to a method for preparing a lyophilizate of the aqueous formulation and to a method for determining the absolute amount of a target protein using the formulation.

### BACKGROUND

Mass spectrometry (MS)-based proteomics is a powerful tool for absolute quantification and sensitive detection of proteins in human samples. In MS, proteins are analysed by their mass and charge. For robust and accurate quantification, stable isotopic variants of the analyte(s) may be used as internal standards. Labelling peptides and proteins with heavy isotope-labeled amino acids has become a common strategy to enable accurate protein quantification. An internal standard has at least one identical sequence as the target protein and carries stable isotopic labels, such as ¹³C, ¹⁵N and ¹⁸O, which are used to introduce a detectable increase in mass. The internal standards serve as markers for proteins of interest and are added to allow the peptides and the proteins they represent to be quantified.

Typical applications of absolute protein quantifications are the determination of cellular copy numbers of proteins and the concentration of biomarkers in body fluids, which is important for several medical applications.

Several methods for absolute quantification have emerged over the last years, including AQUA, QConCAT, PSAQ, SILAC and FlexiQuant. They all quantify the endogenous protein of interest by the heavy to light ratios to a defined amount of the labeled counterpart spiked into the sample. The methods are primarily distinguished from each other by either spiking in heavy labeled peptides or heavy labeled full-length proteins.

Due to difficulties in producing large numbers of heavy isotope-labeled full-length proteins, synthetic peptides with incorporated heavy isotopes are commonly used as internal standards.

EP2694556 discloses the use of a heavy isotope labeled fusion polypeptide as internal standard in a method for determining the absolute amount of a target polypeptide in a sample. The fusion polypeptide comprises at least one tag sequence and a subsequence of the target polypeptide.

The use of isotope labeled fusion polypeptides as internal standards provides a very accurate quantification of a target protein in quantitative mass spectrometry-based assays.

However, such assays are negatively impacted by the lack of proper procedures for storing and handling the internal standard fusion polypeptides. The process for preparing isotope labeled fusion polypeptide standards is often fragile and small deviations from the standard production procedure can cause large problems. Storage in various types of buffers can render the peptides susceptible to microbial growth and degradation. The peptides may lose solubility, and changes in concentration may occur. Another challenge is their susceptibility to chemical denaturation. Proteins can undergo both chemical and physical degradation. Chemical degradation refers to degradation pathways where a protein is modified due to a chemical reaction while physical degradation includes conformation changes, adsorption, denaturation, precipitation and aggregation. Chemical degradation often results in changes of the protein conformation and thereby also physical degradation mechanism such as aggregation and precipitation. Furthermore, the excipients used in a formulation may affect the tendency of a protein to interact with a surface, and protein adsorption to surfaces such as tubes and vials may be a problem during protein storage.

To minimize detrimental effects, isotope labelled internal standards may be formulated as lyophilized or freeze-dried products.

Although the protein stability in a solid state product is better than for the corresponding product in aqueous formulation, chemical denaturation may still be a problem. Chemical stability of lyophilized proteins and peptides may be affected by the excipients used in a formulation. Various types of buffers, polymers, surfactants, amino acids, chelating complexes, and inorganic salts are often used as excipients for freeze-dried biological products. It should be noted that some of these excipients may in fact be detrimental to the stability of the proteins. For example urea is a commonly used buffer ingredient which may result in denaturation of the protein.

In view of this, it is desirable to provide a stable formulation which protects the isotope labeled fusion polypeptide standards during storage both in aqueous and lyophilized conditions, and wherein the functionality of the internal fusion polypeptide standard is maintained after lyophilization. Such a formulation should be suitable for MS sample preparation, and should also provide for stable recovery of the peptides, when reconstituted in water or other solutions after storage.

### SUMMARY

In view of above-mentioned and other drawbacks of the prior art, it is an object of the present disclosure to provide improvements in relation to internal standard fusion polypeptide formulations, particularly with respect to assurance of the quality (concentration, stability) of the fusion polypeptide internal standards in lyophilized form and in solution over time, during both storage and handling.

According to an aspect of the present disclosure, it is therefore provided an aqueous formulation comprising:
(a) 5-50 µM of an isotope labeled fusion polypeptide
(b) HCl in an amount to yield a pH of 7.5-8.5
(c) 0.01-0.5 M of a Tris buffer
(d) 2-20 % (w/w) of a sugar selected from trehalose and sucrose.

In embodiments, the aqueous formulation comprises 8-20 µM of the isotope labeled fusion polypeptide.

This concentration is particularly suitable for protein quantification. If the concentration of the isotope labeled fusion polypeptide is too high, the polypeptide may precipitate in the solution. However, if it is too low, a large volume of the formulation may be required, particularly if a target protein of high concentration is to be quantified.

In embodiments, the aqueous formulation comprises 0.05-0.3 M of the Tris buffer, preferably 0.08-0.15 M.

As mentioned hereinbefore, protein denaturation may be a result of changes in pH, salt concentration, temperature, lyophilization and excipients used together with the isotopically labeled internal standard. It is a challenge to find excipients suitable for a great variety of proteins and peptides, and there is not one formulation that suits all peptides. The formulation according to the present disclosure is particularly suitable for fusion polypeptides, and provides for stable recovery of the peptides in the formulation. The formulation is suitable for storage both in solution and in the form of a lyophilized product. The excipients of the formulation protect the protein structure from collapsing due to osmotic pressure and stress brought during lyophilization procedures.

The pH of the formulation should be in the range of from 7.5 to 8.5, preferably from 7.8 to 8.2 in order to prevent chemical degradation of the peptides. pH affects oxidation of proteins. Proteins can undergo oxidation as a result of molecular oxygen present during both production and storage. Oxidation can occur both in solution and in lyophilized state. For instance, pH affects the reaction by changing the potential of the oxidants. High concentrations of salt can cause precipitation by neutralizing the charge on the protein surface. This is a result of interactions between the protein and the salt molecules instead of the solvent water. Due to the same phenomenon, proteins with an isoelectric point (pI) close to the pH of the surrounding solvent often precipitate due to neutralization of the protein interactions. It is therefore advantageous to have a pH that is distinguished from the pI of the peptides in the formulation. A pH in the range of 7.5-8.2 is higher than the pI of the peptides utilized and is selected since lower pH can catalyze the denaturation process of the peptides.

Trehalose and sucrose stabilize the isotope labelled peptides by affecting the viscosity and surface forces of water and by increasing the molecular density of the solution, which also leads to reduced aggregation.

Sugars and polyols are often used for stabilization of proteins both in liquid and lyophilized state. Commonly used polyols in protein formulations are glycerol and sorbitol, which serve to stabilize proteins. However, during lyophilization, sorbitol can destabilize proteins due to crystallization

Preferably, the aqueous formulation according to the present disclosure comprises 2-20 % (w/w), e.g. 5-15 % (w/w) trehalose.

The use of trehalose in combination with the other ingredients of the formulation according to the invention is advantageous since it improves the stability of the fusion polypeptides in solution and also prevents denaturation of the fusion polypeptides during lyophilization.

As used herein, the term "polypeptide" refers to a polycondensate of amino acids, preferably of the 20 standard amino acids. The term "polypeptide" also covers peptides and proteins (at least to the extent such proteins consist of a single chain).

A "fusion polypeptide" according to the present disclosure is a polypeptide which comprises at least two segments of different origin. More specifically, a fusion polypeptide according to the present disclosure comprises a tag amino acid sequence and a subsequence of the target polypeptide comprised or suspected to be comprised in the recited sample. It is deliberately envisaged that more than one tag amino acid sequence is present.

The term "subsequence" refers to any partial sequence of a target polypeptide to be detected and also includes the entire sequence of the target polypeptide. In preferred embodiments, the subsequence is a partial sequence of the target polypeptide, the entire sequence of said target polypeptide being excluded.

The use of a fusion polypeptide is beneficial since it improves the accuracy in quantitatively determining cellular protein levels. Another advantage is that fusion polypeptides typically yield several quantifiable peptides for each fusion polypeptide, which allows for both accurate quantification of the standard and for absolute quantification of the target polypeptide. Furthermore, production of the internal standard can be streamlined because protein expression can be performed in a standard system (such as E. coli), and a large number of fusion polypeptides can be produced under similar conditions.

The formulation according to the present disclosure provides improvements with respect to handling and storage of the fragile isotope labeled fusion polypeptides. The formulation is suitable for a variety of internal standard fusion polypeptides, and protects the protein structure during storage. Furthermore, the formulation provides for stable recovery of the fusion polypeptides upon reconstitution.

In embodiments, the fusion polypeptides comprises 50-500 amino acids, preferably 150-300 amino acids.

The aqueous formulation according to the present disclosure is particularly suitable for such fusion polypeptides, which may be fragile and subject to degradation. The formulation provides for stable recovery of the fusion polypeptides in the formulation. The excipients of the formulation protect the protein structure in solution, and also during lyophilization by preventing the peptides from collapsing due to osmotic pressure and stress brought during lyophilization.

In embodiments, all the C and N atoms of the Lys and Arg residues of the isotope labeled fusion protein are ¹³C and ¹⁵N, respectively.

The term "isotope" refers to two or more nuclides with the same number of protons (atomic number) but different numbers of neutrons. Such difference in mass number provides for different peak positions of an isotope labeled compound or fragment on the one hand and its unlabeled counterpart on the other hand in a mass spectrum.

Isotope labeling may be achieved by chemical addition of labeled reagents, enzymatic isotope labeling or metabolic labeling. Preferably, the isotope labeling is introduced by metabolic labeling. In other words, the fusion polypeptides of the invention are preferably obtained by means of production in biological systems, such as cell-free as well as cellular systems. For example, a host cell may be used which is auxotrophic for lysine and/or arginine, wherein at the same time isotope labeled lysine and/or arginine is provided in the growth medium. A preferred means of metabolic isotope labeling is stable isotope labeling with amino acids in cell culture (SILAC). As mentioned above, to the extent isotope labeling makes use of isotopes with higher mass numbers, the labeled form is commonly referred to as "heavy" form, whereas the naturally occurring counterpart or the counterpart which is free or essentially free of the heavy isotope under consideration is commonly referred to as "light" form.

In embodiments, the fusion polypeptide comprises a fragment of a target protein and at least one solubility and/or purification tag.

The solubility tag promotes protein solubility, and may be selected from Maltose-binding protein (MBP), Glutathione-S-transferase (GST), Thioredoxin (Trx), N-Utilization substance (NusA), Small ubiquitin-modifier (SUMO), a Solubility-enhancing tag (SET), a Disulfide forming protein C (DsbC), Seventeen kilodalton protein (Skp), Phage T7 protein kinase (T7PK), Protein GB1 domain (GB1), Protein A IgG ZZ repeat domain (ZZ) and Albumin Binding Protein (ABP).

The solubility tag may also be used as a quantitation tag ("tag sequence"). The solubility tag may comprise 40 to 150 amino acids.

Preferably, the solubility tag is an albumin binding protein (ABP) tag.

The ABP tag improves the solubility of the fusion polypeptide and provides for accurate quantitation.

The purification tag may be selected from His tag, a FLAG tag, a SBP tag, a myc tag and a OneStrep tag.
In embodiments, the purification tag is a His tag.

A His tag, or polyhistidine tag is a string of histidine residues at either the N or C terminus of a recombinant protein. A string may comprise 4 to 10 residues, but commonly six histidine residues are used; a hexahistidine tag. His-tag purification uses the purification technique of immobilized metal affinity chromatography (IMAC), where transition metal ions are immobilized on a resin matrix using a chelating agent. The His-tag has a high affinity for the metal ions used (e.g. Ni²⁺, Co²⁺, Cu²⁺, and Zn²⁺) and binds strongly to the IMAC column. The use of a His tag and IMAC can provide relatively pure recombinant proteins directly from a crude lysate.

In embodiments, the fragment of a target protein is a proteotypic peptide subsequence of a target subsequence comprising 15-205 amino acids, preferably 50-150 amino acids.

As used herein, the term "proteotypic peptide subsequence" refers to peptide sequence that is found in only a single known protein and therefore serves to identify that protein. The "proteotypic peptide subsequence" uniquely identifies each protein within a sample mixture.

An isotope labeled fusion polypeptide comprising such a target subsequence is particularly suitable for MS based protein quantification, and provides for accurate and absolute quantification of proteins of low abundance.

Preferably, the fusion polypeptide comprises a proteotypic peptide subsequence of a target subsequence comprising 15-205 amino acids, preferably 50-150 amino acids, an albumin binding protein (ABP) tag and a His tag.

In preferred embodiments, the fusion polypeptide comprises:
- an N terminal quantification tag comprising an albumin binding protein (ABP) tag and a His tag
- a C terminal proteotypic subsequence of a target sequence comprising 15-205 amino acids, preferably 50-150 amino acids.

The ABP and His tags may be collectively be referred to as a quantification tag (Qtag), and may be used for both affinity purification, and accurate quantification of the fusion polypeptide.

The C terminal subsequence of a target protein to be identified may be refererred to as protein epitope signature tag antigen (PrEST antigen or PrEST fragment). The fusion polypeptide according to the embodiment described above may be referred to as QPrEST and is available from Atlas Antibodies. A vast number of QPrESTs have been derived from the Human Protein Atlas project, and are currently available for more than 13000 human proteins

The subsequence of a target protein (the PrEST fragment) preferably comprises at least two unique tryptic peptides. This way, multiple data points from a single standard may be generated (i.e.the generated heavy-to-light peptide ratios can be verified by data from additional tryptic peptides).

The subsequence of a target protein (PrEST fragment) is selected to have minimal sequence identity to other proteins, and excludes signal peptides and/or excludes sequences from transmembrane spanning regions.

Quantification of QPrEST may be performed in an MS based set-up through quantitative analysis of QTag derived tryptic peptides, using unlabeled QTag protein as an internal reference. High purity and an accurately determined concentration of the unlabeled QTag protein is ensured through multiple affinity-purification steps and subsequent amino acid analysis. This targeted QPrEST quantification results in a highly accurate product concentration.

QPrEST internal standards are fragile protein fragments and subject to degradation and variations in concentrations in solution and due to lyophilization. The excipients used in the formulation according to the present disclosure protect the protein structure of the internal standard, and assure the quality thereof, both in lyophilized form and in solution. The storage and handling procedures are significantly improved.

According to another aspect of the present disclosure, there is further provided a method of preparing a lyophilisate, which is intended to be dissolved in water and used in an MS-based quantification of a target protein in a sample, the method comprising a step of lyophilizing the aqueous protein formulation as described above.

According to another aspect, a lyophilized formulation prepared according to the method above is provided.

A significant advantage of the formulation of the present disclosure is that the formulation may be lyophilized without impairing the protein structure of the fusion polypeptide.

When the formulation has been lyophilized; it may be stored more than two years prior to reconstitution in water or other solution.

In embodiments, the concentration of the isotope labeled fusion polypeptide is 0.1-1 mg/ml, e.g. 0.1-0.5 mg/ml.

This allows for accurate MS based quantification of proteins of very low abundance in a biological sample.

According to another aspect of the present disclosure, there is further provided a method for determining the absolute amount of a target protein in a sample comprising:
a) adding an aqueous formulation as described above to a biological sample
b) proteolytically digesting the mixture of step a)
c) subjecting the proteoloytical digest obtained in step b) to mass spectrometric analysis.

As used herein, the term "biological sample" means a sample comprising cells and/or body fluids. The cells may be of a single type or of various types and may be embedded in one or more tissues. The sample may comprise one or more body fluids, such as blood, blood serum, blood plasma, cerebrospinal fluid, mucus, peritoneal fluid, pleural fluid, saliva, semen, sweat, tears and urine.

The biological sample may or may not be proteolytically digested before the internal standard formulation is added. Preferably, the sample has not been digested prior to mixture with the formulation of the invention.

The formulation comprising the isotope labeled fusion polypeptide is added to an unknown sample, and the mixture is thereafter subject to proteolysis, typically by using a proteolytic enzyme such as trypsin. The presence of the isotope labeled fusion polypeptide internal standard during trypsin digestion reduces the potential bias resulting from incomplete cleavage by the proteolytic enzyme, which is often a problem for peptide standards. The presence of endogenous cleavage sites in the fusion polypeptide (QPrEST) enables the use of peptides containing missed cleavage sites, as they usually generate heavy-to light (H/L) ratios similar to those of the corresponding fully cleaved peptides. Peptides containing missed cleavage sites have H/L ratios very similar to those of the fully cleaved peptides. Including these peptides in the analysis can generate additional quantitative information, further increasing the reliability of the analysis. The high correlation between data from different peptides - both fully cleaved peptides and peptides containing missed cleavage sites - indicates that the digestion efficiency is similar for the fusion polypeptide standard and the endogenous protein. This is of great importance and is one of several advantages of using larger protein-fragment standards as opposed to peptide standards, with which incomplete cleavage results in quantitative errors.

The fractionated sample and internal standard are analyzed in a mass spectroscopic device, and the ratio of intensities between a labeled peak and an unlabeled peak is used to calculate the absolute amount of the target protein (and internal standard).

Any type of mass spectrometry based procedure may be used, and the method is not limited to a specific equipment or analysis used. Any equipment with the intent of analyzing the m/z ratio of a sample can be used.

The formulation according to the present disclosure is preferably added to the sample at an early stage of the sample-preparation workflow, decreasing the potential for quantitative errors introduced by, for example, variations in the proteolytic reaction.

The formulation comprising the isotope labeled fusion polypeptide can be used to determine absolute quantities of target proteins in human cell lines with both single-plex and multiplex approaches.

In embodiments, the method further comprises the step of subjecting the proteolytical digest obtained in step b) to chromatographic separation.

The separation procedure is performed to separate the isotope labeled internal standards and the corresponding target peptides from other peptides in the sample. The method is not limited to a specific type of chromatography process, but any suitable means for separation of the peptides may be used. Examples include high-pressure liquid chromatography (HPLC), Reverse Phase-High Pressure Liquid Chromatography (RP-HPLC), electrophoresis (e.g., capillary electrophoresis), anion or cation
exchange chromatography, and open-column chromatography.

Further features of, and advantages with, the present disclosure will become apparent when studying the appended claims and the following description. The skilled addressee realizes that different features of the present disclosure may be combined to create embodiments other than those described in the following, without departing from the scope of the present disclosure.

### BRIEF DESCRIPTION OF THE DRAWINGS

The various aspects of the present disclosure, including its particular features and advantages, will be readily understood from the following detailed description and the accompanying drawings, in which:
Figure 1 schematically illustrates the workflow for the quantification of a target protein of interest using the formulation according to the invention.
Figure 2 illustrates a schematic workflow for accurate determination of PrEST concentrations.

### DETAILED DESCRIPTION

The present disclosure will now be described more fully hereinafter with reference to the accompanying drawings, in which currently preferred embodiments of the present disclosure are shown. The present disclosure may, however, be embodied in many different forms and should not be construed as limited to the embodiments set forth herein; rather, these embodiments are provided for thoroughness and completeness, and fully convey the scope of the present disclosure to the skilled person.

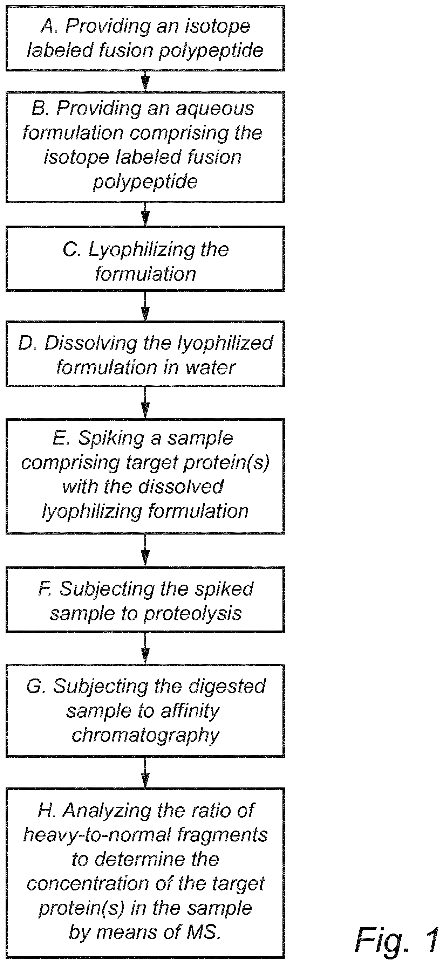

Figure 1 schematically illustrates the workflow for a quantifying a target protein of interest.

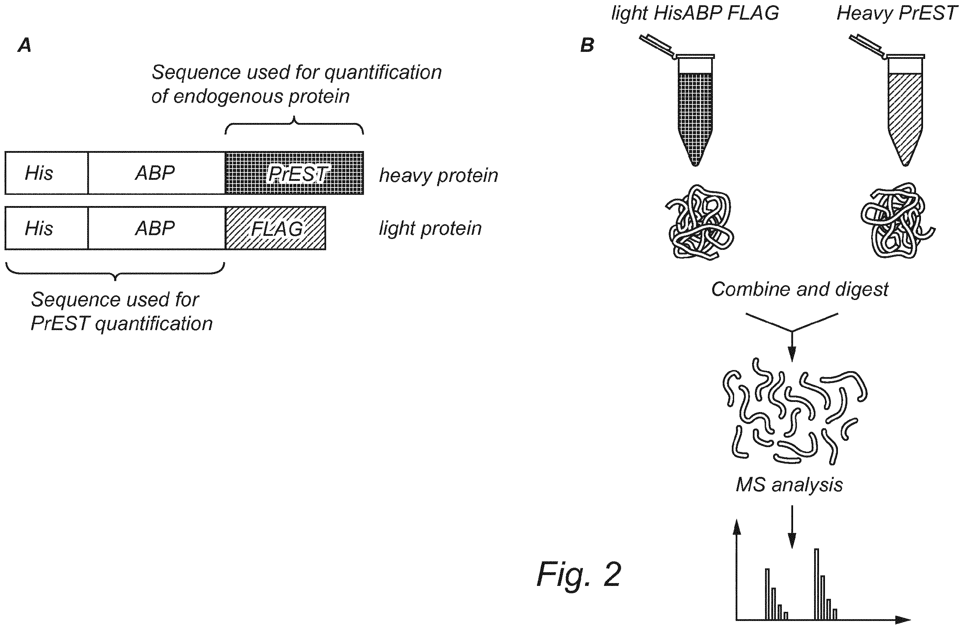

First, an isotope labeled fusion polypeptide is provided (step A). A preferred fusion polypeptide is a recombinant polypeptide comprising a protein epitope signature tag (PrEST) and a HisABP quantitation tag (Q tag), used for purification and quantification (see figure 2a). The PrEST part matches a fragment of the target protein and is chosen to show as low sequence similarity as possible to other proteins. Each PrEST sequence preferably contains multiple tryptic peptides and may therefore give several tryptic peptides, thereby yielding several peptide ratios (heavy/light) for each target protein. This is beneficial for the subsequent quantification.

In the production of the fusion polypeptide PrESTs, a modified E.coli strain, auxotrophic for arginine and lysine is used. Lysine and arginine are supplied to a minimal culture medium in heavy isotope-labeled forms to generate heavy isotope-labeled protein fragments. Lysis and purification is performed according to procedures known in the art. After purification, PrEST quantification is performed using the quantification standard His6ABPFLAG (The Q-tag also including a C-terminal FLAG tag). Peptides originating from the His6ABP region are used for accurate PrEST quantification. Peptides originating from the PrEST sequence can thereafter be used to quantify endogenous proteins in e.g. a cell lysate or blood (figure 2a). The workflow for PrEST quantification is illustrated in figure 2b. PrEST and quantification standard are mixed and digested. Heavy to light ratios from the His6ABP region are used to determine the PrEST concentration.

When mixing unlabeled His6ABPFLAG and isotope labeled PrEST(s) and digesting them with trypsin, peptides originating from the His6ABP tag will be present in both unlabeled and heavy isotope-labeled forms. Since the absolute quantity of the light peptides originating from His6ABPFLAG is known, the obtained heavy to light ratios can be used for determination of the absolute quantity of the heavy isotope-labeled PrEST.

Accurately quantified PrESTS can then be used as internal standards in MS based absolute quantification setups where peptides from the PrEST region are compared to peptides from the corresponding endogenous protein in for example a cell lysate.

After purification and quantification of the isotope labeled fusion polypeptide, an aqueous formulation is provided (step B), which formulation comprises
(a) 5-50 µM of an isotope labeled fusion polypeptide
(b) HCl in an amount to yield a pH of 7.5-8.5
(c) 0.03-0.5 M of a Tris buffer
(d) 2-20 % (w/w) of a sugar selected from trehalose and sucrose

The formulation may then be either used directly in an MS based quantification assay or it may be lyophilized (step C), and the details on such lyophilization is elucidated in the Example section.

If the formulation is a lyophilizate, it is dissolved in water, preferably ultra pure sterile water, prior to use in MS based quantification analysis (step D).

A sample comprising a target protein of interest is spiked with the dissolved (aqueous) lyophilized formulation (step E).

The spiked sample is thereafter subjected to proteolysis (step F) by using a proteolytic enzyme, such as trypsin. The addition of the formulation comprising the isotope labeled fusion protein is preferably performed early in the workflow, which reduces the errors that can occur during sample preparation. This is beneficial compared to many of the other labeled standards that are typically added later in the workflow. This decreases the potential for quantitative errors introduced by e.g. variations in the proteolytic cleavage reaction.

Optionally, the quantification method comprises subjecting the proteolytically digested peptides to chromatographic separation (step G) to separate the target peptides and internal standard peptides from other peptides present in the sample.

The cleaved peptides are thereafter subject to MS analysis (step H). Peptides are, compared to full-length proteins, more easily ionized in the mass spectrometer, and may therefore yield an increased sensitivity. The concentration of the target peptide(s) may be calculated from the relative peak intensities of the isotopically distinct peptides.

### Experimental data

### Determination of protein stability after lyophilization

Two different formulations comprising an isotopically labelled fusion polypeptide; QPrEST31325 (supplied by Atlas Antibodies), were compared to determine the impact of lyophilization on the protein stability and structure.
Sample 1: QPrEST, 1xPBS, 5% (w/w) trehalose
Sample 2: QPrEST 0.1 M Tris-HCL, 5% (w/w) trehalose

After purification, the QPrEST was formulated in 0.1 M Tris-HCL, 5 % Trehalose by a buffer exchange step using Sephadex G-25 desalting columns, supplied by GE Healthcare. The IMAC purification eluate was loaded onto the column and the QPrEST was thereafter eluted by addition of the formulation buffer (0.1 M Tris-HCl, 5% Trehalose). The tris buffer was prepared by first resolving Tris-(hydroximetyl) aminometan ≥99.9% (VWR) to a concentration of 0.1 M in distilled H₂O followed by addition of D-(+)-Trehalose dihydrate from Saccharomyces cerevisiae, ≥99% (Sigma Aldrich) to a concentration of 5 %. The pH was adjusted to 8.0 by addition of HCL and the buffer was thereafter sterilized by using a 0.45 µm bottle top filter.

The QPrEST formulated in the Tris buffer was transferred to low bind tubes in aliquots of 100 µL prior to the lyophilization step. After lyophilization the dried sample was reconstituted by addition of 100 µL distilled H₂O followed by vortexing for 30 s and centrifugation at 4000 x g for 1 min.

### Evaluation of protein stability after lyophilization

Freeze-drying cycle was performed using an Epsilon 2-4 LSCplus (Martin Christ Gmbh, Germany) freeze dryer. The liquid samples were loaded on shelf at 20°C, and the shelf was cooled down to -45°C at 0.36°C/min, frozen at -45°C for 2 h, followed by ramping the shelf to 4°C at 0.41 °C/min at 0.1 mbar, and then keeping the shelf at 4 °C for 18 h. Secondary drying (or final drying) was done at 0.001 mbar by first ramping the shelf to 20°C at 0.27°C/min, and then keeping the shelf at at 20°C for 4 h.

After finishing the freeze drying cycle, the vials were removed from the freeze-dryer and put into a desiccator. Then, the vials were filled with nitrogen gas before closure with a vial lock, and then kept in the desiccator.

The visual appearance of the inventive formulation (sample 2) compared to sample 1 was a lot better. Sample 1 collapsed from top to bottom and looked like a clump, whereas the inventive formulation (sample 2) had a superior, well maintained structure.

### Evaluation of glass transition temperature (Tg) using differential scanning calorimetry (DSC)

The differential scanning calorimeter was carried out using DSC 1 STARe instrument (Mettler-Toledo, Switzerland) to measure glass transition (Tg) and evaluations of the thermograms were done in Star Software v.10.X. About 3-5 mg of the lyophilized sample was weighted in an aluminium pan which with a closed lid and an empty aluminium pan was used as a reference. The sample was kept at 20°C for 2 min before the temperature was reached to 250°C with a heating rate of 10 °C/min under nitrogen gas with the flow rate of 50 ml/min. All the measurements were done in duplicate.

The glass transition temperature of the solutions was measured using DSC prior the freeze-drying cycle to make sure that the sublimation temperature was lower to avoid collapse of the structure of the dry material. The Tg of solutions was measured to be -39°C for tris-5% trehalose and -43°C for PBS-5% trehalose.

DCS measurements were done to evaluate the glass transition temperature for the lyophilized sample 1 and 2 (see results in table 1 below).

### Determination of water content by thermogravimetric analysis (TGA)

Thermogravimetric analyses were performed using Mettler Toledo thermogravimetric analyzer, TGA2. About 3-5 mg of the lyophilized sample was weighted in a sample pan. The temperature was increased from 25 °C to 250 °C with a rate of 20 °C/min under nitrogen gas with the flow rate of 50 ml/min. The sample was kept at the initial temperature for 1 min and for 5 min at the final temperature. The water content of sample 1 and sample 2, respectively, is illustrated in table 1 below. The water content as a function of temperature is illustrated in figure 4.

**Table 1: Glass transition and water content of samples**

| **Sample** | **Tg (°C)** | **Water content** |
|---|---|---|
| Sample 1 | 66 | 2.8 |
| Sample 2 | 112 | 0.53 |

### Conclusion

The glass transition temperature for the formulation according to the invention (sample 2) was significantly higher than for sample 1, which reflects the stability of the protein formulation during storage and transport. Furthermore, the water content was lower for sample 2, which indicates that the inventive formulation is more suitable for long term storage. Furthermore, as mentioned hereinbefore, a visual inspection of the structures of the samples after lyophilization showed that the inventive formulation (sample 2) had a well maintained structure, whereas sample 1 collapsed from top to bottom and looked like a clump.

### Reconstitution of lyophilized inventive formulation

An investigation of the variation between lyophilized QPrEST vials was performed. Nine lyophilized vials of each QPrEST were reconstituted at three different days and quantified by MS at three different days (standard QC).

The samples were reconstituted by addition of 100 µL distilled H₂O followed by vortexing for 30 seconds and centrifugation for 1 min at 4000 x g.

In addition, one vial of each QPrEST batch stored in solution was included and quantified together with the lyophilized samples. Generated concentrations were compared and the variation between vials was determined. Summarized results can be seen in table 2 below. The variation in concentration between lyophilized QPrEST vials was low (mean CV = 4 %), and all QPrESTs showed a variation between vials below 10 %. Only QPrESTs with concentration > 5 µM were included.

In addition to the evaluation of variation also the protein recovery after lyophilization was determined by comparing the median concentration of lyophilized vials to the concentration of the QPrEST samples that were not lyophilized prior to the quantification. The evaluation of the results showed that the protein recovery was more than 85% for all the included QPrESTs.

**Table 2. Variation in concentration between lyophilized QPrEST vials**

| ***OPrEST-ID*** | ***Experiment*** | ***Median concentration (µM) n=9*** | ***CV (%) n=9*** | ***Recovery (%)*** | ***Passed QC criteria?*** |
|---|---|---|---|---|---|
| *OPrEST23910* | *1* | *21.7* | *1* | *89* | *Yes* |
| *OPrEST24495* | *1* | *11.3* | *5* | *90* | *Yes* |
| *OPrEST24496* | *1* | *11.0* | *5* | *89* | *Yes* |
| *OPrEST24526* | *1* | *13.0* | *4* | *107* | *Yes* |
| *OPrEST22218* | *2* | *24.8* | *5* | - | *Yes* |
| *OPrEST22536* | *2* | *5.2* | *11* | - | *Yes* |
| *QPrEST22668* | *2* | *16.3* | *3* | *94* | *Yes* |
| *OPrEST23910* | *2* | *27.2* | *2* | *92* | *Yes* |
| *QPrEST24495* | *2* | *15.5* | *3* | *94* | *Yes* |
| *OPrEST24496* | *2* | *7.5* | *3* | *94* | *Yes* |
| *QPrEST24547* | *2* | *14.1* | *3* | *99* | *No* |

The results from the first part of the investigation of variation study showed that the suggested QC strategy seems to generate acceptable results in terms of precision. One more experiment was performed using the same QPrESTs that were included in the first experiment together with 5 additional QPrESTs, all formulated in 0.1 M Tris-HCl, 5% Trehalose.

Results from experiment 2 further verified that the variations between lyophilized QPrEST vials were generally low (mean CV of all included QPrESTs was 5 %) and fulfill the suggested criterium of 10 %. The analysis also showed that the CV was normally lower for QPrESTs with higher concentrations (mean CV for QPrESTs with concentration higher than 5 µM was 4 %) and only one QPrEST showed a CV above 10% (11% for QPrEST22536).

Protein recovery after lyophilization was investigated for 5 of the QPrESTs included in experiment 2 and results showed that all QPrESTs had a recovery of minimum 90%. The minimum recovery determined by this experiment was higher than what was seen by the first experiment and this is probably due to the generally higher concentrations of the QPrESTs included in part 2.

If summarizing data generated from QPrESTs from both the experiments and that passed the concentration criterium of 5 µM, 90% of the QPrESTs studied passed the suggested variation criteria of 10 %. This data verifies that it is sufficient to use the proposed strategy of performing the MS quantification on a lyophilized aliquot when performing QC of the QPrEST product.

Even though the present disclosure has been described with reference to specific exemplifying embodiments thereof, many different alterations, modifications and the like will become apparent for those skilled in the art.

Variations to the disclosed embodiments can be understood and effected by the skilled addressee in practicing the present disclosure, from a study of the drawings, the disclosure, and the appended claims. Furthermore, in the claims, the word "comprising" does not exclude other elements or steps, and the indefinite article "a" or "an" does not exclude a plurality.

## Claims

1. An aqueous formulation comprising:
(a) 5-50 µM of an isotope labeled fusion polypeptide
(b) HCl in an amount to yield a pH of 7.5-8.5
(c) 0.03-0.5 M of a Tris buffer
(d) 2-20 % (w/w) of a sugar selected from trehalose and sucrose

2. The aqueous formulation according to claim 1, comprising 8-20 µM of said isotope labeled fusion polypeptide.

3. The aqueous formulation according to claim 1 or claim 2, comprising 0.05-0.3 M of said Tris buffer, preferably 0.08-0.12 M.

4. The aqueous formulation according to any one of the preceding claims, comprising 2-20 % (w/w), preferably 5-15 % (w/w) trehalose.

5. The aqueous formulation according to any one of the preceding claims, wherein said fusion polypeptide comprises 50-500 amino acids, preferably 150-300 amino acids.

6. The aqueous formulation according to any one of the preceding claims, wherein all the C and N atoms of the Lys and Arg residues of the isotope labeled fusion protein are ¹³C and ¹⁵N, respectively.

7. The aqueous formulation according to any one of the preceding claims, wherein the fusion polypeptide comprises a fragment of a target protein and at least one solubility and/or purification tag.

8. The aqueous formulation according to claim 7, wherein the solubility tag is an albumin binding protein (ABP) tag.

9. The aqueous formulation according to claim 7 or claim 8, wherein the purification tag is a His tag.

10. The aqueous formulation according to any one of claims 7-9, wherein said fragment of a target protein is a proteotypic subsequence of a target subsequence comprising 15-205 amino acids, preferably 50-150 amino acids.

11. The aqueous formulation according to any one of claims 1-10, wherein said fusion polypeptide comprises:
- an N terminal quantification tag comprising an albumin binding protein (ABP) tag and a His tag
- a C terminal proteotypic subsequence of a target sequence comprising 15-205 amino acids, preferably 50-150 amino acids.

12. A method of preparing a lyophilizate, which is intended to be dissolved in water and used in an MS-based quantification of a target protein in a sample, said method comprising a step of lyophilizing the aqueous protein formulation according to any one of claims 1-11.

13. A lyophilized formulation prepared according to the method of claim 12.

14. A method for determining the absolute amount of a target protein in a sample comprising:
a) adding an aqueous formulation according to any one of claims 1-11 to a biological sample
b) proteolytically digesting the mixture of step a)
c) subjecting the proteoloytical digest obtained in step b) to mass spectrometric analysis.

15. A method according to claim 14, further comprising the step of subjecting the proteolytical digest obtained in step b) to chromatographic separation.
